# EUROPÄISCHE PATENTSCHRIFT

(11) **EP 2 451 774 B1**
(45) Veröffentlichungstag und Bekanntmachung des Hinweises auf die Patenterteilung: **22.07.2015**
(21) Anmeldenummer: 10729809.3
(22) Anmeldetag: 26.06.2010
(51) Int. Cl.: C07C 263/10

(54) **VERFAHREN ZUR HERSTELLUNG VON ISOCYANATEN IN DER GASPHASE**
METHOD FOR PRODUCING ISOCYANATES IN THE GAS PHASE
PROCÉDÉ POUR PRÉPARER DES ISOCYANATES EN PHASE GAZEUSE

(30) Priorität: 09.07.2009 DE 102009032414
(43) Veröffentlichungstag der Anmeldung: 16.05.2012
(73) Patentinhaber: Bayer Intellectual Property GmbH, 40789 Monheim (DE)
(72) Erfinder: BRUNS, Rainer, 51373 Leverkusen (DE); LORENZ, Wolfgang, 41540 Dormagen (DE); SOMMER, Knut, 47804 Krefeld (DE)
(74) Vertreter: BIP Patents
(86) Internationale Anmeldenummer: PCT/EP2010/003915
(87) Internationale Veröffentlichungsnummer: WO 2011/003531

(56) Entgegenhaltungen:
- EP-A1- 2 060 560
- WO-A1-2007/028715
- WO-A1-2009/027232

## Beschreibung

Die Erfindung betrifft ein Verfahren zur kontinuierlichen Herstellung von Isocyanaten durch Umsetzung der korrespondierenden Amine mit Phosgen in einem Reaktor in der Gasphase in Gegenwart mindestens einen inerten Stoffes, worin dem Reaktor ein Phosgen enthaltender Strom und ein das Amin und den inerten Stoff enthaltender Strom zugeführt werden, wobei das molare Verhältnis des inerten Stoffs zu den Aminogruppen in dem Strom keinen großen Schwankungen unterliegt.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Eine Möglichkeit der Herstellung von Isocyanaten ist die Umsetzung der Amine mit dem Phosgen in der Gasphase. Diese üblicherweise als Gasphasen-Phosgenierung bezeichnete Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Hilfsstoffe, Produkte und Reaktionszwischenprodukte, bei den gewählten Bedingungen gasförmig sind. Vorteile der Gasphasen-Phosgenierung sind u. a. ein verringerter Phosgen-Hold-Up, die Vermeidung schwer phosgenierbarer Zwischenprodukte und erhöhte Reaktionsausbeuten. Die vorliegende Erfindung betrifft ausschließlich die Gasphasen-Phosgenierung.

Es sind aus dem Stand der Technik verschiedene Verfahren zur Herstellung von Isocyanaten durch Umsetzung von Aminen mit Phosgen in der Gasphase bekannt.

EP-A-289 840 beschreibt die Herstellung von Diisocyanaten durch Gasphasen-Phosgenierung, wobei die Herstellung in einer turbulenten Strömung bei Temperaturen zwischen 200 °C und 600 °C in einem zylindrischen Raum ohne bewegte Teile stattfindet. Gemäß der Lehre von EP-A-289 840 ist es für die Durchführbarkeit des in der EP-A-289 840 offenbarten Verfahrens wesentlich, dass die Abmessungen des Rohrreaktors und die Strömungsgeschwindigkeiten im Reaktionsraum so bemessen werden, dass in dem Reaktionsraum eine turbulente Strömung herrscht, die gemäß der Lehre von EP-A-289 840 durch eine Reynoldszahl von mindestens 2500 charakterisiert wird. Nach der Lehre von EP-A-289 840 ist diese Turbulenz im Allgemeinen dann gewährleistet, wenn die gasförmigen Reaktionspartner den Reaktionsraum mit einer Strömungsgeschwindigkeit von mehr als 90 m/s durchlaufen. Durch die turbulente Strömung im zylindrischen Raum (Rohr) wird, wenn man von Fluidelementen in Wandnähe absieht, eine relative gute Strömungsgleichverteilung im Rohr und damit eine relativ enge Verweilzeitverteilung erreicht, die, wie in EP-A-570 799 (s. u.) beschrieben, zu einer Verringerung der Feststoffbildung führt. Weiterhin wird der Einsatz der Amine in verdünnter Form offenbart, wobei als Verdünnungsmittel Inertgase

(vorzugsweise Stickstoff) oder Dämpfe inerter Lösungsmittel (wie Chlorbenzol, Dichlorbenzol, Xylol, Chlomaphthalin oder Decahydronaphthalin) beschrieben werden. Die Schrift beschreibt, dass eine Verdünnung unter Einhaltung eines Volumenverhätlnisses von Diamindampf zu Inertgas bzw. Lösemitteldampf von 1:0.5 bis 1:2 erfolgen kann. Allerdings verneint die Schrift ausdrücklich, dass die eingesetzte Menge an Verdünnungsmittel eine kritische Rolle spielen könnte (S. 3, Spalte 3, Z. 46 - 48).

EP-A1-570 799 betrifft ein Verfahren zur Herstellung von aromatischen Diisocyanaten, dadurch gekennzeichnet, dass die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen sowohl zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6 % beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder einer Bodenstein-Zahl von oberhalb 100 charakterisiert wird. Wird die Rohrströmung durch eine Reynoldszahl oberhalb 4000 charakterisiert, ist nachteilig auch hier, dass wegen der notwendigen hohen Strömungsgeschwindigkeiten die Realisierung der zur vollständigen Umsetzung der Amine notwendigen Verweilzeit nur in sehr langen Misch- und Reaktorrohren möglich ist. Weiterhin wird die Möglichkeit der Verdünnung des Amins offenbart, wobei die gleichen Verdünnungsmittel wie in EP-A-289 840 beschrieben werden. Es wird lediglich ein typisches Volumenverhältnis von Verdünnungsmittel zu Diamin im Bereich von 1 : 0,5 bis 1 : 2 beschrieben. Welche Konsequenzen ungünstige Verhältnisse von Verdünnungsmittel zu Amin haben können, ist nicht Gegenstand der Lehre von EP-A-570 799. Nach der Lehre von EP-A-570 799 führen somit bereits kleine Abweichungen von der mittleren Kontaktzeit zu einer unverwünschten Feststoffbildung und verkürzter Reaktorstandzeit.

EP-B-699 657 beschreibt ein Verfahren zur Herstellung von aromatischen Diisocyanaten in der Gasphase, dadurch gekennzeichnet, dass die Umsetzung des zugehörigen Diamins mit dem Phosgen in einem zwei Zonen enthaltenden Reaktor stattfindet, wobei die erste Zone, die etwa 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Bevorzugt wird die zweite Reaktionszone als Rohrreaktor ausgeführt. Weil jedoch mindestens 20 % des Reaktionsvolumens ideal rückvermischt sind, resultiert eine ungleichmäßige Verweilzeitverteilung, die zu einer unerwünschten erhöhten Feststoffbildung führen kann. Weiterhin wird der Einsatz des Amins in Konzentrationen von 3 bis 30 % in einem Verdünnungsmittel of-fenbart (in Beispiel 2 *ortho*-Dichlorbenzol). Welche Konsequenzen ungünstige Verhältnisse von Verdünnungsmittel zu Amin haben können, ist nicht Gegenstand der Lehre von EP-B-699 657.

Die Optimierung des Einsatzes von Rohrreaktoren für die Gasphasen-Phosgenierung, wie grundlegend in EP-A-570 799 unter Anwendung des Strahlmischerprinzips (Chemie-Ing.-Techn. 44 (1972) S. 1055, Abb. 10) offenbart, ist Gegenstand zahlreicher Anmeldungen.

Nach der Lehre der EP-A-1 362 847 haben eine Vergleichmäßigung des über den Ringraum des Rohrreaktors zugeführten Eduktstromes und eine möglichst zentrale Zuführung beider Eduktströme in den Rohrreaktor einen großen positiven Einfluss auf die Stabilität der Reaktionszone und damit auf die Gasphasenreaktion insgesamt. Als Folge der stabileren Reaktionsführung nehmen die beobachteten Temperaturschwankungen deutlich ab, die ohne die offenbarten Maßnahmen zu beobachtende Asymmetrie in der Temperaturverteilung verschwindet praktisch vollständig. Nach der Lehre von EP-A-1 362 847 führen Temperaturschwankungen und Asymmetrien in der Temperaturverteilung zur Bildung von Nebenprodukten, die zu Anbackungen und Verstopfungen im Reaktor und damit zu einer Verkürzung der Standzeit der Reaktoren führen. Spezielle Hinweise zur Umsetzung des offenbarten Verfahrens in einem technischen Maßstab werden in der der EP-A-1 362 847 jedoch nicht offenbart. Weiterhin wird der optionale Einsatz eines Verdünnungmittels (Intertgase oder Dämpfe inerter Lösungsmittel) offenbart, ohne dass die Anmeldung auf Details hinsichtlich einzuhaltender Mengenströme und -verhältnisse eingehen würde.

Eine Weiterentwicklung des Einsatzes von Rohrreaktoren für die Gasphasen-Phosgenierung, wie grundlegend in der EP-A-570 799 unter Anwendung des Strahlmischerprinzips (Chemie-Ing.-Techn. 44 (1972) S.1055, Abb.10) offenbart, ist Gegenstand der WO2007/028715**.** WO2007/028715 offenbart ein Verfahren zur Herstellung von Isocyanaten durch Phosgenierung der entsprechenden Amine in der Gasphase in einem Reaktor, dadurch gekennzeichnet, dass der zum Einsatz kommende Reaktor eine Mischeinrichtung und einen Reaktionsraum aufweist. Gemäß der Lehre von WO2007/028715 umfasst der Reaktionsraum im vorderen Bereich den Mischraum, in dem überwiegend die Vermischung der gasförmigen Edukte Phosgen und Amin, gegebenenfalls vermischt mit Inertmedium, stattfindet, was in der Regel begleitet ist vom Einsetzen der Reaktion. Gemäß der Lehre von WO2007/028715 findet im hinteren Teil des Reaktionsraumes dann im Wesentlichen nur noch die Reaktion und höchstens untergeordnet die Vermischung statt. Bevorzugt werden in dem in WO2007/028715 offenbarten Verfahren zur Strömungsrichtung rotationssymmetrische Reaktionsräume eingesetzt, die konstruktiv im Wesentlichen in bis zu vier Längenabschnitte entlang der Längsachse des Reaktors im Verlauf der Strömung zerlegt werden können, wobei sich die Längenabschnitte in der Größe der durchströmten Querschnittsfläche unterscheiden. Nachteilig bei dem offenbarten Verfahren ist die hohe Strömungsgeschwindigkeit von bevorzugt 10 bis 300 m/s, besonders bevorzugt 40 bis 230 m/s, ganz besonders bevorzugt 50 bis 200 m/s, insbesondere mehr als 150 bis 190 m/s und speziell 160 bis 180 m/s, mit der das gasförmige Reaktionsgemisch den Reaktionsraum durchläuft. Wie bereits in EP-A-570 799 beschrieben, ist auf Grund der hohen Strömungsgeschwindigkeiten die Realisierung der zur vollständigen Umsetzung der Amine notwendigen Verweilzeit, speziell bei Einsatz aromatischer primärer Amine, nur in sehr langen Reaktorrohren möglich. Nachteilig ist ebenfalls, dass die Veränderungen in der durchströmten Querschnittsfläche des Reaktionsraumes durch einen in einem Rohrreaktor befindlichen Volumenkörper erzeugt werden, die Umsetzung des offenbarten Reaktoraufbaus in einem technischen Maßstab somit konstruktiv aufwändig ist. Hinsichtlich des optionalen Einsatzes eines Inertmediums offenbart die Anmeldung, dass das Verhältnis der Gasvolumina von Inertmedium zu Amin bzw. zu Phosgen mehr als 0,0001 bis 30, bevorzugt mehr als 0,01 bis 15, besonders bevorzugt mehr als 0,1 bis 5 beträgt. Welchen Einfluss ungünstige Verhältnisse von Inertmedium zu Amin bzw. Phosgen haben können, ist nicht Gegenstand der Lehre von WO2007/028715.

EP-A-1 935 876 beschreibt die Vorteile adiabater Reaktionsführung, die in der Vermeidung von Temperierungsproblemen und höheren Raum-Zeit-Ausbeuten liegen. Auch hier wird der optionale Einsatz eines Inertmediums (Intertgase oder Dämpfe inerter Lösungsmittel) bei der Verdampfung des Amins offenbart, ohne dass die Anmeldung auf Details bzgl. einzuhaltender Mengenströme und -verhältnisse eingehen würde. Die Schrift lehrt, dass sofern für das jeweilige System basierend auf der Starttemperatur, adiabate Temperaturerhöhung, molares Verhältnis der Reaktanden, Verdünnungsgas und eingesetztes Amin eine einmal ermittelte Mindestverweilzeit für die vollständige Reaktion um weniger als 20%, vorzugsweise um weniger als 10% überschritten wird, kann die Bildung von Folge-Reaktionsprodukten weitgehend vermieden werden.

WO 2009/027232 beschreibt ein grosstechnischer Verfahren zur Herstellung von Isocyanaten.

Der Einsatz von inerten Stoffen zur Verdünnung der Edukte Amin und/ oder Phosgen ist somit Stand der Technik. Insbesondere die Verdünnung des Amins ist allgemeine Praxis. Der Grund hierfür besteht darin, dass geeignete inerte Stoffe, häufig Stickstoff, als Schleppmittel wirken und die Verdampfung des Amins erleichtern, wodurch Zersetzungsreaktionen (beispielsweise unter Ammoniakabspaltung) verringert werden. So bewirkt beispielsweise der Zusatz bereits geringer Mengen an Stickstoff zu Toluylendiamin (in der Regel ein Gemisch verschiedener Isomere, im Folgenden summarisch mit TDA abgekürzt) eine signifikante Verringerung der Verdampfungstemperatur. Beispielsweise führt die Zugabe von 4 Gew% Stickstoff eine Verringerung der Verdampfungstemperatur des TDA um ca. 8°C. Somit reichen bereits geringe Mengen eines inerten Gases aus um die Verdampfungstemperatur abzusenken.

Bei dieser Vorgehensweise ist es wichtig, dass das Verhältnis von inerten Stoffen zum zu verdampfenden Amin keinen großen Schwankungen unterworfen ist. Fällt beispielsweise im Falle der Phosgenierung von TDA unter Verwendung von Stickstoff zur Erleichterung der Verdampfung des TDA infolge einer Betriebsstörung der Stickstoffstrom plötzlich ab, führt dies zu einem sprunghaften Anstieg der Verdampfungstemperatur des TDA und einem kurzfristigen Abfall der verdampften TDA Menge. Die hieraus resultierende Instabilität des TDA-Gas-Stroms zum Reaktor hin hat infolge der hohen Geschwindigkeit der Gasphasen-Phosgenierung desaströse Konsequenzen: Durch die Verringerung des TDA-Stroms verringert sich auch der Strom an freigesetztem Chlorwassergasstoff als Reaktionskoppelprodukt kurzfristig sehr deutlich, da aufgrund der Stöchiometrie der Reaktion die 4fache Gasmenge HCl bezogen auf TDA freigesetzt wird, was wiederum, zusammen mit der verringerten Stickstoffmenge, zu einem deutlichen Druckabfall im Reaktor führt. Zudem verändert sich die molare Verhältnis der Reaktanden, und infolge dessen bei einer adiabaten Reaktionsführung der adiabate Temperatursprung. Die nach Lehre von EP-A-570 799 erforderliche geringe Abweichung von nur 6% von der mittleren Kontaktzeit wird nicht eingehalten.

Ebenso hat ein kurzfristig erhöhter Stickstoffstrom dramatische Auswirkungen auf die Reaktion: in diesem Fall verdampft kurzfristig mehr TDA, die Menge an freigesetztem Chlorwasserstoffgas als Reaktionskoppelprodukt steigt kurzfristig stark an, was zu einem kurzfristigen Anstieg des Reaktordruckes führt. Auch in diesem Fall wird das molare Verhältnis der Reaktanden gestört mit Auswirkungen auf den adiabaten Temperatursprung der Reaktion, zudem wird die in EP-A-570 799 erforderliche geringe Abweichung von nur 6% von der mittleren Kontaktzeit nicht eingehalten.

Somit führen sowohl ein abnehmender als auch ein ansteigender Stickstoffstrom zu Störungen Gasphasenphosgenierrreaktion, und zwar durch Störung der angestrebten Stöchiometrie der Reaktanden, was nach Lehre aus dem Stand der Technik zu vermehrter Nebenproduktbildung und Bildung von festen Ablagerung führt, welche die Reaktion empfindlich stören und im schlimmsten Fall eine Abschaltung des Reaktors erforderlich machen können. Die für das System zunächst bestimmt Mindestverweilzeit wird nicht eingehalten, was nach der Lehre aus dem Stand der Technik zur Bildung von Reaktionsfolgeprodukten führt. Aufgrund der Schnelligkeit der Gasphasenphosgenierung verändert sich sehr schnell die Reaktionsflammentemperatur und es kommt zu Temperaturschwankungen, was nach Lehre nach Stand der Technik zur Bildung von Nebenprodukten führt, die zu Anbackungen und Verstopfungen im Reaktor und damit zu einer Verkürzung der Standzeit der Reaktoren führen.

Bei der praktischen Durchführung des Verfahrens kann folglich ein zeitlich nicht stabiles Verhältnis zwischen den inerten Stoffen und dem Edukt eine Abweichung von der mittleren Kontaktzeit bewirken mit den aus dem Stand der Technik negativen Konsequenzen für die Durchführung einer Gasphasenreaktion. Keines der bisher offenbarten Schriften befasst sich mit diesen Problemen; daher ist dem Stand der Technik eine Lösung derselben nicht zu entnehmen.

Aufgabe der vorliegenden Erfindung war es daher, ein Verfahren zur Gasphasen-Phosgenierung von Aminen bereitzustellen, welches sich die Vorteile der Verdünnung des Amins mit inerten Stoffen zunutze macht, ohne dass die oben geschilderten Probleme den Betriebsablauf beeinträchtigen.

Es wurde gefunden, dass die Aufgabe gelöst werden kann durch ein Verfahren zur kontinuierlichen Herstellung von Isocyanaten durch Umsetzung der korrespondierenden Amine mit Phosgen in einem Reaktor in der Gasphase in Gegenwart mindestens einen inerten Stoffes, dadurch gekennzeichnet, dass dem Reaktor ein Phosgen enthaltender Strom und ein das Amin und den inerten Stoff enthaltender Strom zugeführt werden, wobei das molare Verhältnis des inerten Stoffs zu den Aminogruppen in dem Strom
(i) stets > 0 und < 45 mol-%, bevorzugt zwischen 0,05 und 25 mol-%, besonders bevorzugt zwischen 0,1 und 10 mol-% ist und
(ii) sich innerhalb eines Zeitraums von 20 Minuten um maximal 99%, bevorzugt nicht mehr als 80%, besonders bevorzugt nicht mehr als 60 %, ändert, bezogen auf den Zeitpunkt zu Beginn des Zeitraums von 20 min.

Enthält der das Amin und den inerten Stoff enthaltende Strom zwei oder mehr inerte Stoffe, so berechnet sich das molare Verhältnis in (i) und in (ii) mit der Summe der Mole der inerten Stoffe.

Dabei werden bevorzugt primäre Amine eingesetzt. Bevorzugt werden primäre aromatische Amine eingesetzt, die im Wesentlichen ohne Zersetzung in die Gasphase überführt werden können. Irisbesondere bevorzugt sind primäre aromatische Diamine.

Beispiele für bevorzugte aromatische Amine sind Toluylendiamin (TDA), insbesondere 2,4- TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus.

Weiterhin sind besonders primäre Amine, insbesondere primäre Diamine, auf Basis von aliphatischen oder cycloaliphatischen Kohlenwasserstoffen mit 2 bis 18 Kohlenstoffatomen geeignet. Besonders gut geeignete Amine sind 1,6-Diamino-hexan, 1-Amino-3,3,5-trimethyl-5-aminomethylcyclohexan (IPDA) und 4,4'-Diaminodicyclohexylamin.

Bei einem inerten Stoff im Sinne der Erfindung handelt es sich um einen Stoff, der bei der Reaktionstemperatur gasförmig im Reaktionsraum vorliegt und nicht wesentlich mit den im Reaktionsverlauf auftretenden Verbindungen reagiert. Damit ist gemeint, dass weniger als 10 mol-%, bevorzugt weniger als 5 mol-%, besonders bevorzugt weniger als 3 mol-% und ganz besonders bevorzugt weniger als 1 mol-% des inerten Stoffes unter den Reaktionsbedingungen chemisch reagieren. Außerordentlich besonders bevorzugt reagiert ein inerter Stoff im Sinne der Erfindung überhaupt nicht chemisch unter den Reaktionsbedingungen. Als inerte Stoffe kommen beispielsweise einerseits solche Stoffe in Frage, die bereits bei Raumtemperatur gasförmig vorliegen, also beispielsweise Stickstoff, Edelgase wie Helium oder Argon, andere Gase wie Kohlenstoffdioxid oder Kohlenstoffmonoxid. Andererseits kommen als inerte Stoffe auch solche in Frage, die erst bei Temperaturen oberhalb Raumtemperatur gasförmig vorliegen, also beispielsweise Aromaten wie Chlorbenzol, Chlortoluol (o-, m-, p-Isomere), Dichlorbenzol (o-, m-, p-Isomere), Toluol, Xylol (o-, m-, p-Isomere), Chlornaphthalin (alle Isomere) oder Decahydronaphthalin. In der Gruppe der bereits bei Raumtemperatur gasförmig vorliegenden Stoffe ist Stickstoff besonders bevorzugt, weil dieser die Kriterien hinsichtlich chemischer Inertheit äußerst gut erfüllt und dabei wesentlich preisgünstiger ist als Edelgase (die Effekte in ähnlicher Größenordnung ergeben würden und noch geringere chemische Reaktivität haben). In der Gruppe der erst oberhalb Raumtemperatur gasförmige vorliegenden Stoffe sind Stoffe bevorzugt, die auch als Lösemittel im Prozess verwendet werden. Insbesondere bevorzugt sind Chlorbenzol und Dichlorbenzol. Weiterhin lassen sich die inerten Stoffe im Sinne der vorliegenden Erfindung nach ihrem Verbleib in dem Gasphasenverfahren charakterisieren. Stoffe, die bereits bei Raumtemperatur gasförmig vorliegen, wie zum Beispiel Stickstoff, Edelgase wie Helium oder Argon, andere Gase wie Kohlenstoffdioxid oder Kohlenstoffmonoxid werden mit dem gebildeten, gasförmigen Reaktionskoppelprodukt Chlorwasserstoff im wesentlichen gasförmig aus dem Prozess ausgeschleust, und müssen somit stetig frisch zugeführt werden. Stoffe, die erst oberhalb von Raumtemperatur gasförmig vorliegen, werden hingegeben nicht gasförmig mit dem gasförmigen Reaktionskoppelprodukt Chlorwasserstoff ausgeschleust, sondern verbleiben im Prozess und können, ggfs. nach destillativer Reinigung, wieder als inerter Stoff für die Verdünnung des Amins verwendet werden.

Aus welcher der beiden Gruppen inerte Stoffe gewählt werden, hängt von den produktionstechnischen Randbedingungen ab. Ohne besondere Anforderungen aufgrund der produktionstechnischen Gegebenheiten ist in der Regel Stickstoff zu bevorzugen, weil dieser aufgrund seiner geringen Molmasse deutlich größere Effekte hinsichtlich der Herabsetzung der Verdampfungstemperatur des Amins ausübt als Dichlorbenzol, was zur Folge hat, dass die nötigen Ströme an inertem Stoff deutlich geringer ausfallen können. Stellen die weiteren produktionstechnischen Gegebenheiten jedoch besondere Anforderungen, beispielsweise bei der Aufarbeitung von in der Phosgenierung gebildetem gasförmigen Chlorwasserstoff, kann es auch von Vorteil sein, inerte Stoffe aus der Gruppe der Verbindungen, die erst oberhalb Raumtemperatur gasförmig vorliegen, einzusetzen, beispielsweise Dichlorbenzol. Der Chlorwasserstoff kann beispielsweise nach dem literaturbekannten Deacon-Verfahren (WO2004 014845), d.h. oxidativ in Gegenwart eines Katalysators, zu Chlor aufgearbeitet werden. Wird der Chlorwasserstoff nach dem Deacon-Verfahren zu Chlor aufgearbeitet, welches zumindest partiell wieder für die Herstellung von Phosgen durch Umsetzung von Kohlenmonoxid mit Chlor verwendet werden kann, kann es, abhängig von den genauen Mengenströmen an inertem Stoff, vorteilhaft sein, einen kondensierbaren inerten Stoff wie Dichlorbenzol einzusetzen, da in diesem Fall die Ausschleusung von Stickstoff aus dem Phosgenierprozess in den Deacon Prozess entfällt. Wenn in dem gasförmigen Chlorwasserstoffstrom aus einem Isocyanatprozess größere Mengen Stickstoff enthalten sind, so wird dadurch in dem Deacon Prozess das Wertprodukt HCl Gas verdünnt, damit die Gasbelastung der Apparate erhöht, was zu höheren Apparatekosten führt. Zudem ist die Ausschleusung der Stickstoffmengen aus einem Chlorwasserstoffstrom in dem Deacon Prozess mit nicht unerheblichem Aufwand verbunden und erhöht die Betriebskosten. Ist jedoch die eingesetzte Menge an inertem, bereits bei Raum-temperatur gasförmig vorliegenden Stoff nicht zu groß, kann es auch bei Einsätz eines Deacon-Verfahrens zur Aufarbeitung des gebildeten Chlorwasserstoffs vorteilhaft sein, Stickstoff oder andere bei Raumtemperatur gasförmige inerte Stoffe einzusetzen. Zusätzlich zu den weiter oben geschilderten Problemen, die Schwankungen im Inertgasstrom auf die Verdampfung des Amins haben, kommt in dein Fall der sich an die Isocyanatproduktion anschließenden oxidativen Chlorwassserstoff Rückgewinnung zu Chlor nach dem Deacon Verfahren noch hinzu, dass Schwankungen im Inertgasstrom auch die Aufarbeitung des Chlorwasserstoffs im Deacon Prozess erschweren, sodass die Anwendung des erfindungsgemäßen Verfahrens hier ganz besonders vorteilhaft ist.

Daher wird in einer bevorzugten Ausführungsform des erfindungsgemäßen Verfahrens zusätzlich
(iii) das in der Umsetzung von Phosgen und Amin gebildete HCl zumindest teilweise in einem Deacon Verfahren zu Chlor umgesetzt.

Insbesondere kann es bevorzugt sein, einerseits die Vorteile des Zusatzes einer inerten Verbindung auf die Aminverdampfung in einer Gasphasenphosgenierung auszunutzen, ohne dadurch den Nachteil der Verdünnung des Reaktionskoppelproduktes Chlorwasserstoffgas in der Umsetzung des Chlorwasserstoffgases in einem Deacon Prozess zu Chlor erhalten. In diesem Fall wird bevorzugt soviel inerter Stoff zugesetzt, dass
(iv) das molare Verhältnis des mit dem Amin enthaltenden Strom in den Reaktor eingeführten inerten Stoffs zu dem in das Deacon-Verfahren geführten HCl stets zwischen > 0 und 25 mol-%, bevorzugt zwischen > 0 und 11 mol%, besonders bevorzugt zwischen 0,025 und 5 mol-% liegt.

Gemäß dem erfindungsgemäßen Verfahren erfolgt die Umsetzung von Phosgen mit den aromatischen Aminen in der Gasphase. Unter Umsetzung in der Gasphase ist dabei zu verstehen, dass die Reaktionsbedingungen so gewählt werden, dass die Edukte, Reaktionszwischenprodukte und Produkte sowie zudosierte inerte Stoffe im Verlauf der Umsetzung während des Durchgangs durch den Reaktionsraum überwiegend, insbesondere zu ≥ 95 Massen-%, bevorzugt zu ≥ 98 Massen-%, besonders bevorzugt ≥ 99 Massen-%, ganz besonders bevorzugt ≥ 99,8 Massen-% und speziell zu ≥ 99,9 Massen-%, jeweils bezogen auf die Masse des Reaktionsgemisches, in der Gasphase verbleiben. Zwischenprodukte sind dabei beispielsweise bei Einsatz von Diaminen gebildete Mono-amino-monocarbamoylchloride, Dicarbamoylchloride, Monoamino-monoisocyanate und Monoisocyanato-monocarbamoylchloride sowie die Hydrochloride der jeweiligen Aminoverbindungen.

Die erfindungsgemäße Umsetzung von Phosgen mit aromatischen Aminen erfolgt in zumindest einem Reaktionsraum, der in einem Reaktor angeordnet ist, d. h. unter Reaktionsraum wird der Raum verstanden, in dem die Umsetzung der Edukte und Zwischenprodukte erfolgt, unter Reaktor wird die technische Vorrichtung verstanden, die den Reaktionsraum enthält. Ein Reaktor kann dabei auch mehrere Reaktionsräume enthalten. Dem Reaktionsraum werden im Allgemeinen die Edukte sowie die zudosierten inerten Stoffe über zumindest eine Mischeinrichtung zugeführt. Das erfindungsgemäße Verfahren ist prinzipiell auf jede Reaktionsraum- und Reaktorgeometrie anwendbar.

In einer weiteren bevorzugten Ausführungsform des erfindungsgemäßen Verfahrens weist der Reaktor im Anschluss an den Reaktionsraum, in dem nach der Mischung der Edukte ein Umsatz der Amingruppen zu den Isocyanatgruppen von 80 %, bevorzugt 90 %, besonders bevorzugt 99 %, ganz besonders bevorzugt 99,5 % erzielt wird, einen rotationssymmetrischen Reaktionsraum mit konstanter und / oder erweiterter durchströmter Querschnittsfläche auf.

Das erfindungsgemäße Verfahren ist prinzipiell auf jede Fahrweise anwendbar. Bevorzugt ist die in EP-A-1 935 876 beschriebene adiabate Fahrweise. Das beschriebene Verfahren ist jedoch auch in einer isothermen Fahrweise vorteilhaft, da sich trotz der isothermen Fahrweise, die zum Beispiel durch Kühlung der exothermen Reaktion von außen erreicht werden kann, sich aufgrund der Schnelligkeit der Gasphasenreaktion eine sehr schnelle Änderung der Reaktortemperatur ergibt, die aufgrund der Trägheit des Kühlmittelsystem nicht ausreichend schnell kompensiert werden kann, so dass es auch im Fall einer isothermen Fahrweise der Reaktion zu Temperaturschwankungen im Reaktor mit dem im Stand der Technik beschriebenen Nachteilen kommt.

Die Ausgangsamine werden bevorzugt vor der Durchführung der Phosgenierung verdampft und auf 200 °C bis 600 °C, bevorzugt 200 °C bis 500 °C, besonders bevorzugt 250 °C bis 450 °C erhitzt und, verdünnt mit mindestens einem inerten Stoff gemäß obiger Definition, dem Reaktionsraum zugeführt.

Bevorzugt wird der inerte Stoff der Verdampfung zugeführt, insbesondere bevorzugt wird der inerte Stoff an der Stelle der Verdampfung zugeführt, an der das Amin zumindest ganz oder teilweise von der flüssigen in die gasförmige Phase überführt wird. Es ist jedoch auch prinzipiell denkbar den inerten Stoff in der Verdampfung hinter der Position der zumindest partiellen Überführung des Amins von der flüssigen in die gasförmige Phase einzuspeisen. Weiterhin ist es möglich, das Amin zunächst in einem geeigneten Behälter mit dem inerten Stoff zu mischen, damit zu beaufschlagen oder den inerten Stoff einperlen zu lassen und den so erhaltenden Strom in die Verdampfung zu führen.

Inerte Stoffe die bei Raumtemperatur bereits gasförmig vorliegen, werden bevorzugt gasförmig der Verdampfung zugeführt. Inerte Stoffe die erst oberhalb von Raumtemperatur gasförmig vorliegen, können sowohl flüssig als auch gasförmig, d.h. nach vorheriger Verdampfung in den Aminverdampfung eingebracht werden. Es ist jedoch auch möglich, die inerten Stoffe, die erst oberhalb von Raumtemperatur gasförmig sind, zunächst ggf. in einem geeigneten Behälter mit dem Amin im gewünschten Verhältnis zu mischen und den so erhaltenden Strom gemeinsam zu verdampfen.

Sofern der zugeführte inerte Stoff bei Raumtemperatur gasförmig ist, wird er bevorzugt mit einer Temperatur von -30°C bis 600°C, bevorzugt 0 bis 500°C, insbesondere bevorzugt 50 bis 400°C zu der Verdampfung zugeführt. Zur Erreichung dieser Temperatur sind verschiedene Apparate wie zum Beispiel Rohrbündelwärmetauscher mit Kühl- oder Heizmittel oder elektrisch betriebene Erhitzer oder sonstige Apparate geeignet. Insbesondere bevorzugt hat der zugeführte bei Raumtemperatur gasförmige inerte Stoff eine Temperaturdifferenz zum Siedepunkt des Amins bei dem gegebenen Verdampfungsdruck von nicht mehr als 150°C, bevorzugt nicht mehr als 100°C.

Sofern der zugeführte inerte Stoff erst oberhalb der Raumtemperatur gasförmig ist, wird er, sofern er gasförmig zugeführt wird, zunächst verdampft und bevorzugt mit einer Temperatur von 200°C bis 600°C, insbesondere bevorzugt mit einer Temperatur von 250°C bis 500°C der Aminverdampfung zugeführt. Insbesondere bevorzugt hat der zugeführte gasförmige erst oberhalb von Raumtemperatur gasförmige inerte Stoff eine Temperaturdifferenz zum Siedepunkt des Amins bei dem gegebenen Verdampfungsdruck von nicht mehr als 150°C, bevorzugt nicht mehr als 100°C.

Zur Erzeugung eines gasförmigen inerten Stroms eines erst oberhalb von Raumtemperatur gasförmig vorliegenden Stoffes sind alle dem Fachmann bekannten Apparaturen geeignet.

Sofern der erst oberhalb von Raumtemperatur gasförmige Stoff zunächst mit dem Amin vermischt wird, kann die Temperatur des inerten Stoffes weit variieren. Möglich ist ein Temperaturbereich von minus 30°C bis 200°C und bevorzugt 0 bis 180°C. Möglich ist auch die erhaltene Mischung aus Amin und erst oberhalb von Raumtemperatur gasförmigen inerten Stoff gemeinsam zu erwärmen und als Mischung in die Verdampfung zu führen. Es ist aber auch möglich beide Ströme getrennt zu erwärmen und erst in der Verdampfung zu vereinen.

Möglich ist es prinzipiell auch Mischungen aus verschiedenen inerten Stoffen einzusetzen. Vorteilhaft können Mischungen aus bei Raumtemperatur bereits gasförmig vorliegenden Stoffen und erst oberhalb von Raumtemperatur gasförmig vorliegenden Stoffen sein. Dieses kann insbesondere bevorzugt sein, wenn auf der einen Seite der Vorteil einer erniedrigten Verdampfungstemperatur mit einer hohen Reinheit des gasförmigen Koppelproduktes Chlorwasserstoffgas verbunden werden soll.

Eine erfindungsgemäße ausreichende Konstanz im Verhältnis der Ströme wird bevorzugt durch eine konstante und gleichbleibende, bevorzugt zeitlich gleichbleibende Mischung der Ströme erreicht. Insbesondere wird eine ausreichende Konstanz im Verhältnis der Ströme erreicht, indem der massenmäßig kleinere der Ströme, d.h. der zugeführte inerte Stoff, in konstanter Menge und zeitlich stabil zugeführt wird.

Geeignete Maßnahmen, um bei Raumtemperatur bereits gasförmig vorliegende inerte Verbindungen konstant und zeitlich stabil zuzuführen sind dem Fachmann bekannt. Hier sei beispielsweise jedoch nicht beschränkend genannt, dass der Gasdruck des zugeführten gasförmigen Stromes ausreichend hoch ist, um eine geregelte und stabile Zuführung zu ermöglichen. So ist bevorzugt der Druck des zugeführten inerten Stoffes in der Zuführung, d.h. in der Zuführleitung unmittelbar vor Eintritt in den Verdampfer, höher als der Druck der in der Verdampfungsapparatur herrscht. Insbesondere bevorzugt ist der Druck mindestens 10 mbar, ganz besonders bevorzugt mindestens 50 mbar, und insbesondere bevorzugt mindestens 150 mbar höher als der Druck in der Verdampfungsapparatur. Im Allgemeinen ist der Druck nicht mehr als 100 bar höher. Insbesondere bevorzugt werden gasförmig zugeführte inerte Stoffe gemessen und geregelt zugerührt. Geeignete Messinstrumente und Regelinstrumente sind Stand der Technik. Andere Maßnahmen sind ebenfalls möglich.

Sofern als inerter Stoff eine Substanz, die erst oberhalb von Raumtemperatur gasförmig ist, verwendet wird, und dieser Stoff vor der Verdampfung flüssig mit dem Amin vermischt, wird die ausreichende Konstanz im Verhältnis der Ströme durch Vermischung von flüssigen Strömen erreicht. Hierzu wird bevorzugt der massenmäßig kleinere der Ströme, d.h. der inerte Strom, in konstanter Menge und zeitlich stabil zugeführt wird. Geeignete Maßnahmen flüssige Ströme konstant zu dosieren sind dem Fachmann bekannt. Eine geeignete Dosierung in konstanter Menge und zeitlich stabil kann zum Beispiel mit einer Dosierpumpe erfolgen, es ist aber auch die Einfüllung in einen Behälter und Vermischung zum Beispiel mittels Rührer denkbar. Andere Maßnahmen sind ebenfalls möglich.

Bevorzugt wird durch diese Maßnahmen sichergestellt, dass sich der Molstrom an inertem Stoff bzw. die Summe der Molströme aller inerter Stoffe innerhalb eines Zeitraums von 20 Minuten um nicht mehr als 99 %, bevorzugt nicht mehr als 80 %, besonders bevorzugt nicht mehr als 60 % ändert. Hierdurch werden starke Schwankungen im Verhältnis der Summe der Molströme aller inerten Stoffe zum Molstrom der Amingruppen vermieden, sodass sich dieses Verhältnis innerhalb eines Zeitraums von 20 Minuten um nicht mehr als 99 %, bevorzugt nicht mehr als 80 %, besonders bevorzugt nicht mehr als 60 % ändert. Infolgedessen werden die oben beschriebenen Probleme im Zusammenhang mit Störungen im Inertgasstrom effektiv reduziert bzw. ganz vermieden. Durch die Einhaltung eines molaren Verhältnisses des inerten Stoffs zu den Aminogruppen in dem das Amin und den inerten Stoff enthaltenden Strom zum Reaktor von > 0 und < 45 mol-%, bevorzugt von > 0 bis 25 mol-% und besonders bevorzugt von 0,1 bis 10 mol-% wird eine schonende Verdampfung des Amins gewährleistet.

Bevorzugt wird durch diese Maßnahmen zudem sichergestellt, dass sich Schwankungen in der Zusammensetzung im Massestrom des gasförmigen Reaktionskoppelprodukts Chlorwasserstoff vermieden werden. Durch die so zeitlich stabilisierte Zusammensetzung des gasförmigen Chlorwasserstoffstromes werden Störungen in dem Deacon Prozess vermieden.

Die Verdampfung der Ausgangsamine kann in allen bekannten Verdampfungsapparaturen erfolgen. Bevorzugt werden Verdampfungssysteme eingesetzt, bei denen ein kleiner Arbeitsinhalt mit einer hohen Umwälzleistung über einen Fallfilmverdampfer geführt wird, wobei zur Minimierung der thermischen Belastung der Ausgangsamine der Verdampfungsvorgang unter Zuspeisung von mindestens einem inertem Stoff unterstützt wird.

In einer besonders bevorzugten Ausführungsform werden Verdampfungssysteme eingesetzt, bei denen ein kleiner Arbeitsinhalt über zumindest einen Mikrowärmeaustauscher oder Mikroverdampfer umgewälzt wird. Der Einsatz entsprechender Wärmeaustauscher für die Verdampfung von Aminen wird z. B. in EP-A-1 754 698 offenbart. Bevorzugt werden in dem erfindungsgemäßen Verfahren die in den Absätzen [0007[ bis [0008] und [0017] bis [0039] der EP-A-1 754 689-offen-barten Apparate eingesetzt.

Das verdampfte Gemisch aus Amin und mindestens einem inerten Stoff kann noch Anteile an unverdampften Tröpfchen an Amin enthalten und im Fall der Verwendung solcher inerter Stoffe, die erst oberhalb Raumtemperatur gasförmig vorliegen, u. U. auch noch Anteile an unverdampftem inertem Stoff. Das verdampfte Gemisch aus Amin und inertem Stoff kann also als Aerosol vorliegen. Bevorzugt enthält das verdampfte Gemisch aus Amin und inertem Stoff jedoch im wesentlichen keine Tröpfchen an unverdampftem Amin und / oder unverdampftem inertem Stoff, das heißt maximal 0,5 Massen-%, besonders bevorzugt maximal 0,05 Massen-% des verdampften Gemisches aus Amin und inertem Stoff, bezogen auf die Gesamtmasse des verdampften Gemisches aus Amin und inertem Stoff, liegen in der Form unverdampfter Tröpfchen vor. Der restliche Teil des verdampften Gemisches aus Amin und inertem Stoff liegt dampfförmig vor. Ganz besonders bevorzugt enthält das verdampfte Gemisch aus Amin und mindestens einem inertem Stoff keine Tröpfchen an unverdampften Anteilen. Bevorzugt wird nach der Verdampfung das Gemisch aus Amin und mindestens einem inertem Stoff, über einen Nacherhitzer auf die gewünschte Einsatztemperatur gebracht.

Weiterhin bevorzugt erfolgt die Verdampfung und Überhitzung der Ausgangsamine/Inertstoffe mehrstufig, um unverdampfte Tröpfchen im Gasstrom aus Amin und mindestens einem inertem Stoff zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind z. B. beschrieben in "Droplet Separation", A. Bürkholz, VCH Verlagsgesellschaft, Weinheim - New York - Basel - Cambridge, 1989. Besonders bevorzugt sind Tröpfchenabscheider die einen geringen Druckverlust verursachen. Ganz besonders bevorzugt wird das verdampfte Gemisch aus Amin und mindestens einem inertem Stoff über zumindest einen Nacherhitzer auf die gewünschte Einsatztemperatur gebracht, der auch als Tröpfchenabscheider fungiert. Insbesondere bevorzugt hat dieser Nacherhitzer einen Flüssigkeitsablauf um die stetige Entleerung des Abscheiders zu gewährleisten. Nach Verlassen des in Strömungsrichtung letzten Überhitzers wird das auf seine Solltemperatur vorgeheizte Gemisch aus Amin und mindestens einem inertem Stoff mit einer mittleren Verweilzeit von bevorzugt 0,01 bis 60 s, ganz besonders bevorzugt von 0,01 bis 30 s, insbesondere bevorzugt 0,01 bis 15 s, dem Reaktor bzw. dessen Mischeinrichtung zur Umsetzung zugeführt. Dabei wird über technische Maßnahmen, z. B. eine ausreichende Isolierung zur Vermeidung von Abstrahlungsverlusten, der Gefahr einer erneuten Tröpfchenbildung begegnet. Durch die Erzeugung eines im Wesentlichen tröpfchenfreien Gasstromes aus Amin und mindestens einem inertem Stoff vor Eintritt in den Reaktor wird die Reaktorlaufzeit deutlich erhöht.

In einer bevorzugten Ausführungsform erfolgt die Zuführung des Gasstromes aus Amin und mindestens einem inertem Stoff auf den Reaktor bzw. dessen zumindest eine Mischeinrichtung druckverlustarm ohne Regeleinrichtung. Eine geregelte Zuführung ist jedoch ebenfalls möglich. Möglich ist auch eine Aufteilung des Gasstromes aus Amin und mindestens einem inertem Stoff in mehrere Teilströme, die dann, wie z. B. in EP-A-1 449 826 in den Absätzen [0019] bis [0022] beschrieben, einem Reaktionsraum oder, wie z. B. in WO 2008/055898 S. 8, Z. 25 bis S. 15., Z. 31 und insbesondere S. 23, Z. 19 - 31 beschrieben, mehreren Mischeinrichtungen, zugeführt werden. Bevorzugt erfolgt auch bei einem Aufteilung des Gasstromes aus Amin und mindestens einem inertem Stoff die Zuführung der Teilströme druckverlustarm ohne zusätzliche Regeleinrichtungen. Es ist aber auch eine getrennt geregelte Zuführung der Teilströme möglich.

Beim erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1 : 1 bis 20: 1, bevorzugt 1,2 : 1 bis 5 : 1 vor. Auch das Phosgen wird auf Temperaturen von 200 °C bis 600 °C erhitzt und, optional ebenfalls verdünnt mit mindestens einem inerten Stoff gemäß obiger Definition, dem Reaktionsraum zugeführt.

In einer bevorzugten Ausführungsform erfolgt eine geregelte Zuführung des Phosgenstromes auf den Reaktor bzw. dessen zumindest eine Mischeinrichtung. Eine druckverlustarme Zuführung ohne Regeleinrichtung ist jedoch ebenfalls möglich. Möglich ist auch eine Aufteilung des Phosgenstromes in mehrere Teilströme, die dann, wie z. B. in WO 2008/055898 S. 8, Z. 25 bis S. 15., Z. 31 und insbesondere S. 23, Z. 19 - 31 beschrieben, mehreren Mischeinrichtungen eines Reaktors zugeführt werden. Auch die Zuführung der Teilströme auf mehrere Reaktoren ist möglich. Bevorzugt erfolgt bei einer Aufteilung des Phosgenstromes eine getrennt geregelte Zuführung der Phosgenteilströme.

Das erfindungsgemäße Verfahren wird bevorzugt so durchgeführt, dass die getrennt aufgeheizten Reaktionspartner über zumindest eine Mischeinrichtung in wenigstens einen Reaktionsraum eingeführt, vermischt und unter Beachtung geeigneter Reaktionszeiten unter bevorzugt adiabater Reaktionsführung umgesetzt werden. Anschließend wird durch Abkühlung des Gasstromes das Isocyanat kondensiert, wobei die Abkühlung bis zu einer Temperatur oberhalb der Zersetzungstemperatur des entsprechenden Carbaminsäurechlorids, also beispielsweise Toluylendiaminsäurechlorid im Falle von TDA, erfolgt.

Die notwendige Verweilzeit zur Reaktion der Amingruppen mit dem Phosgen zu Isocyanat liegt zwischen 0,05 und 15 Sekunden, in Abhängigkeit von der Art des eingesetzten Amins, der Starttemperatur, ggf. der adiabaten Temperaturerhöhung im Reaktionsraum, dem molaren Verhältnis von eingesetztem Amin und Phosgen, Art und Menge des mindestens einen inerten Stoffes sowie dem gewählten Reaktionsdruck.

Besonders bevorzugt kommen Reaktoren mit im Wesentlichen rotationssymmetrischen Reaktionsräumen zum Einsatz, bei denen die gasförmigen Edukte und mindestens ein inerter Stoff dem zumindest einen Mischraum nach dem Strahlmischerprinzip (siehe Chemie-Ing.-Techn. 44 (1972) S. 1055, Abb. 10) zugeführt werden. Bevorzugt treten dabei die eingespeisten Stoffströme (d. h. Amin und mindestens ein inerter Stoff einerseits sowie Phosgen, ggf. verdünnt mit inerten Stoffen andererseits) mit einem Geschwindigkeitsverhältnis von 2 - 20, besonders bevorzugt 3 - 15, ganz besonders bevorzugt 4 - 12, in den zumindest einen Mischraum der Reaktoren ein. Bevorzugt wird dabei dem zumindest einen Mischraum der Reaktoren das Gemisch Amin und mindestens einem inertem Stoff mit der höheren Strömungsgeschwindigkeit zugeführt.

Bevorzugt weisen weder der Reaktionsraum noch etwaige Mischaggregate oder Mischräume Heizflächen, die zu einer thermischen Belastung mit der Folge von Folgereaktionen wie Isocyanurät- oder Carbodiimidbildung Anlass geben könnten, oder Kühlflächen auf, die Anlass zu einer Kondensation mit der Folge von Ablagerungen geben könnten. Die Komponenten werden so, abgesehen von etwaigen Abstrahlungs- und Ableitungsverlusten, bevorzugt adiabat umgesetzt. Dabei wird die adiabate Temperaturerhöhung im Mischaggregat und Reaktor bzw. Reaktor allein über die Temperaturen, Zusammensetzungen und relativen Dosierungen der Eduktströme sowie der Verweilzeit in den Mischaggregaten und den Reaktoren eingestellt.

Nach der erfolgten Phosgenierungsreaktion im Reaktionsraum wird bevorzugt das gasförmige Reaktionsgemisch, das bevorzugt wenigstens ein Isocyanat, Phosgen, mindestens einen, bevorzugt genau einen, inerten Stoff sowie Chlorwasserstoff umfasst, von dem gebildeten Isocyanat befreit. Dies kann beispielsweise so erfolgen, dass das den Reaktionsraum kontinuierlich verlassende Reaktionsgemisch, einer Kondensation in einem inerten Lösungsmittel unterzogen wird, wie sie bereits für andere Gasphasen-Phosgenierungen empfohlen wurde (EP-A-0 749 958).

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der eingesetzten Amine und des Phosgens zu den entsprechenden Isocyanaten ein oder mehrere geeignete Flüssigkeitsströme ("Quench-Flüssigkeiten") eingesprüht werden. Dadurch kann, wie in EP-A-1 403 248 beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Zone (Kühlzone) in eine Quenchstufe, wie sie z. B. in EP-A-1 403 248 offenbart wurde, integriert. In einer besonders bevorzugten Form kommen mehrere Kühlzonen zum Einsatz. Integration und Betrieb dieser zumindest zwei Kühlzonen erfolgen bevorzugt mit einer Quenchstufe. Dies ist hinsichtlich Aufbau und Betrieb in EP-A-1 935 875 offenbart.

Statt des integrierten Verbundes von der zumindest einen Kühlzone eines Reaktors mit einer Quenchstufe, wie sie in der EP-A-1 935 875 offenbart wurde, ist ebenfalls der entsprechende integrierte Verbünd der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

In einer bevorzugten Ausführungsform des erfindungsgemäßen Verfahrens beträgt die Durchsatzkapazität des eingesetzten Reaktors bei den erfindungsgemäß geforderten Reaktionsbedingungen > 1 t Amin / h, bevorzugt 2 - 50 t Amin / h, besonders bevorzugt 2 - 12 t Amin / h. Besonders bevorzugt gelten diese Werte für Toluylendiamin, 1,6-Diamino-hexan und für Isophorondiamin. Unter Durchsatzkapazität ist dabei zu verstehen, dass in dem Reaktor die genannte Durchsatzkapazität an Amin pro h umgesetzt werden kann.

Unabhängig von der Art der gewählten Abkühlung wird die Temperatur der zumindest einen Abkühlzone bevorzugt so gewählt, dass sie einerseits oberhalb der Zersetzungstemperatur des dem Isocyanat entsprechenden Carbamoylchlorids liegt und andererseits das Isocyanat und eingesetzte inerte Stoffe, die erst oberhalb Raumtemperatur gasförmig vorliegen, weitestgehend kondensieren bzw. sich weitestgehend in dem Lösungsmittel lösen. Überschüssiges Phosgen, Chlorwasserstoff und inerte Stoffe, die bei Raumtemperatur gasförmig vorliegen, werden in der Kondensations- bzw. Quenchstufe bevorzugt weitestgehend nicht kondensiert bzw. nicht gelöst. Zur selektiven Gewinnung des Isocyanats aus dem gasförmig Reaktionsgemisch besonders gut geeignet sind bei einer Temperatur von 80 bis 200 °C, vorzugsweise 80 bis 180 °C gehaltene Lösungsmittel wie beispielsweise Chlorbenzol und / oder Dichlorbenzol, oder in diesem Temperaturbereichen gehaltenes Isocyanat oder Mischungen des Isocyanats mit Chlorbenzol und / oder Dichlorbenzol. Es ist für den Fachmann aufgrund der physikalischen Daten bei gegebener Temperatur, Druck und Zusammensetzung einfach vorhersagbar, welcher Massenanteil des Isocyanates in der Quenche kondensiert bzw. diese nicht kondensiert durchläuft. Ebenso ist es einfach vorherzusagen, welcher Massenanteil des überschüssigen Phosgens, Chlorwasserstoffs und mindestens einen inerten Stoffes die Quenche unkondensiert durchläuft bzw. sich in der Quencheflüssigkeit löst.

Die Erzeugung der für das erfindungsgemäße Verfahren bevorzugten Strömung des gasförmigen Reaktionsgemischs als Strömung ohne wesentliche Rückvermischung durch den Reaktionsraum wird durch ein Druckgefälle über den Reaktionsraum sichergestellt. Bevorzugt besteht das Druckgefälle zwischen den Eduktzuleitungen vor der Vermischung einerseits und dem Ausgang aus der Kondensations- bzw. Quenchstufe andererseits. Bevorzugt liegt der absolute Druck in den Edukt-Zuleitungen vor der Vermischung bei 200 bis 3000 mbar und hinter der Kondensations- bzw. Quenchstufe bei 150 bis 2500 mbar. Wesentlich ist hierbei jedoch lediglich die Aufrechterhaltung eines Differenzdrucks von den Eduktzuleitungen über den Reaktionsraum bis hinter die Kondensatiöns- bzw. Quenchstufe von bevorzugt mindestens 50 mbar zwecks Gewährleistung der genannten gerichteten Strömung und einer guten Vermischung der Edukte.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit, bevorzugt anschließend in an sich bekannter Weise von überschüssigen Phosgen befreit. Dies kann mittels einer Kühlfalle, Absorption in einem inerten Lösungsmittel (z. B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionraumes eingesetzt.

Die Reindarstellung der Isocyanate erfolgt bevorzugt anschließend durch destillative Aufarbeitung der Lösungen bzw. Gemische aus der Kondensations- bzw. Quenchstufe.

### Beispiele

### Beispiel 1 (Vergleichsbeispiel):

20,5 kmol/h eines Gemisches bestehend aus 2,4 und 2,6-Toluylendiamin im Gewichts-Verhältnis von 80% zu 20% werden zusammen mit 17,85 kmol/h Stickstoff bei ca. 280°C verdampft und gasförmig einem rotationssymetrischen Rohrreaktor mit nachgeschalteter Isocyanatkondensationsstufe mit einer Temperartur oberhalb von 300°C zugeleitet. Gleichzeitig wird parallel dazu gasförmiges Phosgen dem Rohrreaktor ebenfalls mit einer Temperatur von oberhalb 300°C zugeführt. Die Ströme werden durch eine Düse in die Mischzone eingedüst, vermischt und treten in den Reaktionsraum ein. Die Umsetzung in dem Reaktionsraum erfolgt adiabatisch innerhalb einer Verweilzeit von weniger als 10 Sekunden. Das Gasgemisch wird durch eine Kondensationsstufe geleitet und dabei auf eine Gastemperatur von ca. 165 °C abgekühlt. Das anfallende Kondensat wird einer Destillationssequenz zugeführt und ergibt reines TDI. Das nicht kondensierte Gasgemisch wird in einer anschließenden Wäsche mit o-Dichlorbenzol gewaschen und das Nebenprodukt HCl vom überschüssigen Phosgen absorptiv getrennt.

Innerhalb von 8 min fällt die Stickstoffmenge zunächst auf ca. 20% des ursprünglichen Wertes ab, um dann auf ca. 150% des ursprünglichen Wertes anzusteigen. Dadurch fällt die TDA Menge zunächst auf ca. 40% der ursprünglichen Menge ab, um dann auf ca. 125% der ursprünglichen Menge anzusteigen. Dadurch schwankt die mittlere Verweilzeit in dem Reaktor um mehr als +- 10%. Eine Inspektion der Mischdüse zeigt Ablagerungen. Die gemessenen Temperaturschwankungen an der Außenwand auf dem Reaktor betragen +- 3°C.

### Beispiel 2 (erfindungsgemäß):

20,5 kmol/h eines Gemisches bestehend aus 2,4 und 2,6-Toluylendiamin im Gewichts-Verhältnis von 80% zu 20% werden zusammen mit 17,85 kmol/h Stickstoff bei ca. 280°C verdampft und gasförmig einem rotationssymetrischen Rohrreaktor mit nachgeschalteter Isocyanatkondensationsstufe mit einer Temperartur oberhalb von 300°C zugeleitet. Gleichzeitig wird parallel dazu gasförmiges Phosgen dem Rohrreaktor ebenfalls mit einer Temperatur von oberhalb 300°C zugeführt. Die Ströme werden durch eine Düse in die Mischzone eingedüst, vermischt und treten in den Reaktionsraum ein. Die Umsetzung in dem Reaktionsraum erfolgt adiabatisch innerhalb einer Verweilzeit von weniger als 10 Sekunden. Das Gasgemisch wird durch eine Kondensationsstufe geleitet und dabei auf eine Gastemperatur von ca. 165 °C abgekühlt. Das anfallende Kondensat wird einer Destillationssequenz zugeführt und ergibt reines TDI. Das nicht kondensierte Gasgemisch wird in einer anschließenden Wäsche mit o-Dichlorbenzol gewaschen und das Nebenprodukt HCl vom überschüssigen Phosgen absorptiv getrennt.

Innerhalb von 20 min ändert sich die Stickstoffmenge bezogen auf die TDA Gasmenge um nicht mehr als plus / minus 5%. Die mittlere Verweilzeit in dem Reaktor schwankt um nicht mehr als 6%, es werden keine Temperaturänderungen auf der Reaktorwand gemessen. Eine Inspektion der Mischdüse zeigt keine Ablagerungen.

## Patentansprüche

1. Verfahren zur kontinuierlichen Herstellung von Isocyanaten durch Umsetzung der korrespondierenden Amine mit Phosgen in einem Reaktor in der Gasphase in Gegenwart mindestens einen inerten Stoffes, **dadurch gekennzeichnet, dass** dem Reaktor ein Phosgen enthaltender Strom und ein das Amin und den inerten Stoff enthaltender Strom zugeführt werden, wobei das molare Verhältnis des inerten Stoffs zu den Aminogruppen in dem Strom
(i) stets > 0 und < 45 mol-% ist und
(ii) sich innerhalb eines Zeitraums von 20 Minuten um maximal 99 % ändert, bezogen auf den Zeitpunkt zu Beginn des Zeitraums von 20 min.

2. Verfahren nach Anspruch 1, **dadurch gekennzeichnet, dass** als inerter Stoff Stickstoff oder ein oder mehrere Edelgase oder Gemische daraus als inerter Stoff eingesetzt werden.

3. Verfahren nach Anspruch 1, **dadurch gekennzeichnet, dass** als inerter Stoff Chlorbenzol, Chlortoluol, Dichlorbenzol, Toluol, Xylol, Chlornaphthalin oder Decahydronaphthalin oder Gemische daraus als inerter Stoff eingesetzt werden.

4. Verfahren nach Anspruch 1, **dadurch gekennzeichnet, dass**
(iii) das in der Umsetzung von Phosgen und Amin gebildete HCl zumindest teilweise in einem Deacon Verfahren zu Chlor umgesetzt wird.

5. Verfahren nach Anspruch 4, **dadurch gekennzeichnet, dass**
(iv) das molare Verhältnis des mit dem Amin enthaltenden Strom in den Reaktor eingeführten inerten Stoffs zu dem in das Deacon-Verfahren geführten HCl stets zwischen > 0 und 25 mol-% liegt.

6. Verfahren nach Anspruch 1, **dadurch gekennzeichnet, dass** der das Amin und den inerten Stoff enthaltende Strom einem Verdampfer entnommen wird, in dem das Amin verdampft wird und in den der inerte Stoff gasförmig zugeführt wird, wobei der Druck des inerten Stoffs in der Zuführung unmittelbar vor Eintritt in den Verdampfer mindestens 10 mbar höher ist als der Druck im Verdampfer.

7. Verfahren nach Anspruch 1, **dadurch gekennzeichnet, dass** als Amin Toluylendiamin, Diaminohexan oder Isophorondiamin eingesetzt werden.

## Claims

1. Process for the continuous preparation of isocyanates by reaction of the corresponding amines with phosgene in a reactor in the gas phase in the presence of at least one inert substance, **characterized in that** a phosgene-containing stream and a stream containing the amine and the inert substance are fed to the reactor, wherein the molar ratio of the inert substance to the amino groups in the stream
(i) is always > 0 and < 45 mol% and
(ii) within a period of 20 minutes changes by a maximum of 99 %, based on the time at the start of the period of 20 min.

2. Process according to claim 1, **characterized in that** nitrogen or one or more noble gases or mixtures thereof are employed as the inert substance.

3. Process according to claim 1, **characterized in that** chlorobenzene, chlorotoluene, dichlorobenzene, toluene, xylene, chloronaphthalene or decahydronaphthalene or mixtures thereof are employed as the inert substance.

4. Process according to claim 1, **characterized in that**
(iii) the HCl formed in the reaction of phosgene and amine is at least partially converted into chlorine in a Deacon process.

5. Process according to claim 4, **characterized in that**
(iv) the molar ratio of the inert substance introduced into the reactor with the stream containing amine to the HCl fed into the Deacon process is always between > 0 and 25 mol%.

6. Process according to claim 1, **characterized in that** the stream containing the amine and the inert substance is removed from a vaporizer in which the amine is vaporized and into which the inert substance is fed in gaseous form, the pressure of the inert substance in the feed directly before entry into the vaporizer being at least 10 mbar higher than the pressure in the vaporizer.

7. Process according to claim 1, **characterized in that** toluylenediamine, diaminohexane or isophoronediamine are employed as the amine.

## Revendications

1. Procédé pour la production continue d'isocyanates par mise en réaction des amines correspondantes avec du phosgène dans un réacteur dans la phase gazeuse en présence d'au moins une substance inerte, **caractérisé en ce qu'**on envoie au réacteur un courant contenant du phosgène et un courant contenant l'amine et la substance inerte, le rapport molaire de la substance inerte aux groupes amino dans le courant
(i) étant toujours > 0 et < 45 % en moles et
(ii) se modifiant en un espace de temps de 20 minutes d'au maximum 99 %, par rapport à l'instant au début de l'espace de temps de 20 min.

2. Procédé selon la revendication 1, **caractérisé en ce qu'**on utilise comme substance inerte l'azote ou un ou plusieurs gaz rares ou des mélanges de ceux-ci.

3. Procédé selon la revendication 1, **caractérisé en ce qu'**on utilise comme substance inerte le chlorobenzène, le chlorotoluène, le dichlorobenzène, le toluène, le xylène, le chloronaphtalène ou le décahydronaphtalène ou des mélanges de ceux-ci.

4. Procédé selon la revendication 1, **caractérisé en ce que**
(iii) le HCl formé dans la réaction du phosgène et d'amine est au moins en partie converti en chlore dans un procédé Deacon.

5. Procédé selon la revendication 4, **caractérisé en ce que**
(iv) le rapport molaire de la substance inerte introduite dans le réacteur avec le courant contenant l'amine au HCl envoyé dans le procédé Deacon est toujours compris entre > 0 et 25 % en moles.

6. Procédé selon la revendication 1, **caractérisé en ce que** le courant contenant l'amine et la substance inerte est prélevé d'un vaporiseur, dans lequel l'amine est vaporisée et dans lequel la substance inerte est introduite sous forme de gaz, la pression de la substance inerte dans le conduit d'alimentation immédiatement avant l'entrée dans le vaporiseur étant supérieure d'au moins 10 mbar à la pression dans le vaporiseur.

7. Procédé selon la revendication 1, **caractérisé en ce qu'**on utilise comme amine la toluylènediamine, le diaminohexane ou l'isophoronediamine.
